# EUROPEAN PATENT SPECIFICATION

(11) **EP 2 571 977 B1**
(45) Date of publication and mention of the grant of the patent: **08.01.2020**
(21) Application number: 10851869.7
(22) Date of filing: 17.05.2010
(51) Int. Cl.: C12N 1/12, C12M 3/00, C12M 1/00

(54) **SYSTEM FOR SEEDING CELLS ONTO THREE DIMENSIONAL SCAFFOLDS**
SYSTEM ZUR ZELLENAUSSETZUNG AUF DREIDIMENSIONALEN GERÜSTEN
SYSTÈME POUR INOCULER DES CELLULES SUR DES ÉCHAFAUDAGES TRIDIMENSIONNELS

(43) Date of publication of application: 27.03.2013
(73) Proprietor: Yale University, Inc., New Haven CT 06510 (US); Pall Corporation, Port Washington, NY 11050 (US)
(72) Inventor: BREUER, Christopher, Bethany CT 06524 (US); SNYDER, Edward, L., Wallingford CT 06492 (US); SHAFI, Keru, Anaheim Hills CA 92807 (US); SMITH, Martin, Alexander, Reading MA 01867 (US)
(74) Representative: Hoeger, Stellrecht & Partner Patentanwälte mbB
(86) International application number: PCT/US2010/035109
(87) International publication number: WO 2011/146046

(56) References cited:
- WO-A1-91/17809
- US-A- 5 266 480
- US-A- 5 792 603
- US-A- 5 792 603
- US-A- 5 998 184
- US-A1- 2003 093 034
- US-A1- 2004 039 441
- US-A1- 2005 008 626
- US-B1- 6 218 182
- US-B1- 6 416 995
- US-B2- 7 291 450
- US-B2- 7 291 450

## Description

### STATEMENT REGARDING GOVERNMENT SPONSORED RESEARCH

The U.S. Government has certain rights in this invention pursuant to the following grants: 5K08HL083980-05 awarded to Dr. Christopher Breuer by the United States National Institutes of Health (NIH).

### FIELD OF THE INVENTION

The present invention relates to a system, including an apparatus and method for the collection, isolation, seeding of cells from a source, e.g., an individual, directly onto a biocompatible scaffold in preparation for implantation.

### BACKGROUND INFORMATION

Vascular and cardiothoracic surgeons use vascular grafts to repair or replace segments of arterial and venous blood vessels that are weakened, damaged, or obstructed due to trauma or disease such as aneurysm, atherosclerosis, and infection. Historically, vascular grafts have been either homografts, such as the patient's own saphenous vein or internal mammary artery, prosthetic grafts made of synthetic materials such as polyester (e.g., Dacron), expanded polytetraflouroethylene (ePTFE), and other composite materials, or fresh or fixed biological tissue grafts.

However, synthetic grafts generally have inadequate patency rates for many uses, while the harvesting of homografts requires extensive surgery which is time-consuming, costly, and traumatic to the patient. Fixed tissue grafts do not allow for infiltration and colonization by the host cells, which is essential to remodeling and tissue maintenance. Consequently, fixed tissue grafts degrade with time and will eventually malfunction.

Due to the inadequacies of these currently available synthetic and biological grafts, and the high cost and limited supply of homografts, tissue engineered grafts are being developed which are sterilized, then seeded and cultured, in vitro, with cells. These tissue engineered grafts may be superior to other grafts for use in replacement therapy in that they may display the long term dimensional stability and patency of native arteries and vessels with normal physiologic functionality.

Historically, cell isolation and seeding in addition to culturing of tissue-engineered grafts generally requires the use of a sterile environment, such as a hood or specialized facility, such as an ISO Class 7 room. However, there are disadvantages to seeding and culturing tissue in such an environment. For example, these systems can be cumbersome for the user, inconvenient, time-consuming to use, and very expensive to build and maintain.

US patent US 5,792,603 A discloses an apparatus and a method for sterilizing, seeding, culturing, storing, shipping and testing vascular grafts. The apparatus disclosed includes a fluid reservoir, a pump, an alternating pressure source, and at least one treatment chamber. By alternating pressure to a support structure within the treatment chamber upon which a vascular graft scaffold is positioned, a varying radial stress is placed on the scaffold.

US patent US 7,291,450 B2 relates to methods and compositions regarding the preparation of a cell concentrate from a physiological solution, such as bone marrow aspirate.

Thus, there exists a need in the art for compositions and methods that allow for the convenient, sterile isolation, collection and seeding of tissue engineered grafts and other prosthetic devices.

### SUMMARY

Described herein is a system as set out in claim 1. The system, preferably a closed, sterile system, includes an apparatus and methods, for isolating and collecting cells, e.g., bone marrow-derived progenitor cells, such as mononuclear cells, from a source, preferably, a subject, and then seeding them onto a biocompatible, three-dimensional scaffold or tissue graft. The scaffold or graft can be further incubated within the system for culture, transport, storage, testing and/or implantation into a subject, e.g., as a tissue graft, such as a vascular graft, to regenerate and/or repair a tissue *in vivo, in vitro* or *ex vivo.*

In accordance with the present invention, there is provided an apparatus and method for isolating and seeding, culturing, storing, shipping, and testing vascular grafts within a closed and sterile system that does not require a specialized hood or clean room. In certain aspects, the present invention provides an apparatus and method for seeding and culturing vascular grafts with human cells, resulting in a tissue engineered vascular graft populated with viable human cells.

In certain aspects, the invention provides a closed, disposable system that isolates the desired cell type using a filtration/elution technique, and seeds the cells onto a three-dimensional biocompatible scaffold using vacuum seeding. The seeded scaffold can then be incubated and removed from the system when ready for use.

Accordingly, the system for seeding cells is provided comprising a cellular isolate fluid container; a flow channel disposed between the cellular isolate fluid container and a collection fluid container, an elution fluid container, and a seeding container, wherein the flow channel comprises an inlet, an outlet and a filter therebetween, wherein the filter is adapted to allow flow in at least two directions, and wherein the flow channel is selectively in fluid communication with each of the cellular isolate fluid container; the collection fluid container, the elution fluid container, and the seeding container, respectively. In certain embodiments, the seeding container comprises a porous or perforated tube, e.g., a perforated mandril, and a biocompatible three-dimensional scaffold which is apposed to at least a portion of the tube. In certain additional embodiments, the system includes a residual seeded cell fluid container in fluid communication with the seeding container, and a vacuum source in fluid communication with the residual seeded cell fluid container.

In another aspect, methods for using a system for seeding cells as described herein is provided.

The preceding general areas of utility are given by way of example only and are not intended to be limiting on the scope of the present disclosure and appended claims. Additional objects and advantages of the present invention will be appreciated by one of ordinary skill in the art in light of the instant claims, description, and examples.

### BRIEF DESCRIPTION OF THE DRAWINGS

The accompanying drawings, which are incorporated into and form a part of the specification, illustrate several embodiments of the present invention and, together with the description, serve to explain the principles of the invention. The drawings are only for the purpose of illustrating an embodiment of the invention and are not to be construed as limiting the invention. Further objects, features and advantages of the invention will become apparent from the following detailed description taken in conjunction with the accompanying figures showing illustrative embodiments of the invention, in which:

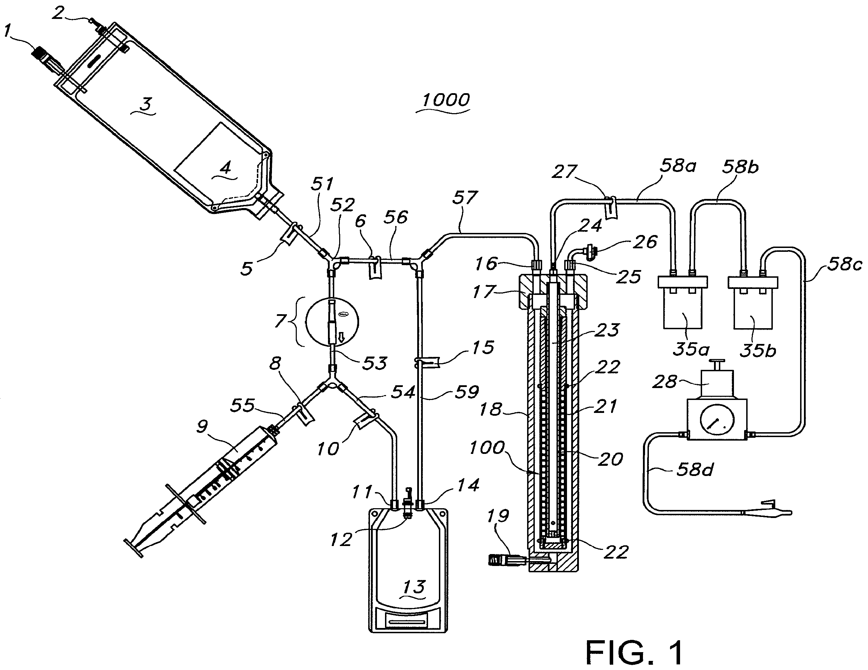

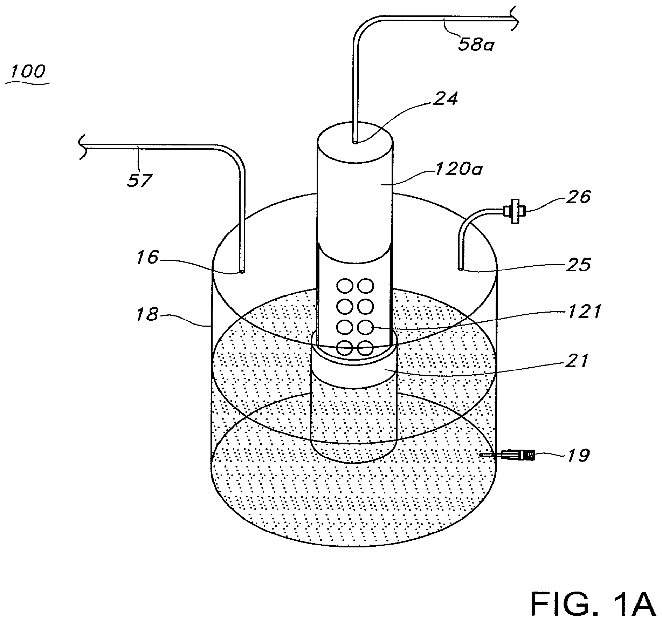

**FIGURE 1** demonstrates an exemplary system as described herein. Briefly, (i) a bone marrow aspirate (e.g., 5 cc/kg body weight) is aseptically collected and injected into cell isolate fluid container (3); (ii) using gravity, the bone marrow aspirate is passed through a flow channel (7) including a filter which traps the bone marrow derived-mononuclear cells as the aspirate passes from the upstream surface of the filter medium and through the downstream surface of the filter medium; (iii) the remaining portion of the bone marrow aspirate (which is typically composed primarily of plasma, but may include red blood cells and/or platelets) is collected in collection fluid container (13); (iv) the elution solution in elution fluid container (9) is passed through the flow channel (7), wherein the elution solution passes from the downstream surface of the filter medium and through the upstream surface of the medium, and into a seeding container (18) such that the filter releases the bone marrow-derived mononuclear cells which are collected in the seeding container; (v) the seeding container (18) contains a scaffold that is inserted over a perforated mandril (20); (vi) the bone marrow-derived mononuclear cell suspension fills the seeding container (18) completely covering the scaffold that is inserted over the perforated mandril (20); (vii) a vacuum (e.g., - 20 mm Hg) is applied until all of the cell suspension has passed through the scaffold and is collected in at least one residual seeded cell fluid container (35a, 35b); and (viii) the filtered bone marrow aspirate, typically primarily comprising plasma, is passed, via gravity, from the collection container (13), into seeding container (18), which contains the seeded scaffold, thus bathing the seeded scaffold.
**FIGURE 1A** is a perspective view of the seeding container (18) shown in **FIG. 1****,** illustrating cell suspension in the seeding container, wherein the suspension contacts the scaffold.
**FIGURE 2** demonstrates an exemplary mandril design for use with an exemplary system as described herein. Briefly, (i) the open end (126) of the perforated mandril (20) is inserted over the suction rod (23); (ii) the appropriate sized coupling rings (123) are then inserted over the perforated mandril; and (iii) the appropriate sized scaffold is then inserted over the apparatus and secured.
**FIGURE 3** illustrates another seeding container (18) that can be used in accordance with embodiments of the invention.

### DETAILED DESCRIPTION

The following is a detailed description of the invention provided to aid those skilled in the art in practicing the present invention.
Unless otherwise defined, all technical and scientific terms used herein have the same meaning as commonly understood by one of ordinary skill in the art to which this invention belongs. The terminology used in the description of the invention herein is for describing particular embodiments only and is not intended to be limiting of the invention.

Described herein is a system, preferably, a closed, sterile system, including an apparatus and methods, for isolating and collecting cells, e.g., bone marrow-derived progenitor cells, such as mononuclear cells, which are seeded onto a biocompatible, three-dimensional scaffold, incubated, and which can then be, for example, implanted, e.g., as a tissue graft, such as a vascular graft. In certain embodiments, the system utilizes a filtration/elution technique, for sterile isolation, seeding, and incubation of cells on to three-dimensional scaffolds. The sterile isolated cells populating the biocompatible scaffold can be cultured and employed in storing, shipping, and/or testing the cells from an individual. Alternatively, the scaffold populated with viable isolated cells can be cultured and used, for example, as an implantable graft, e.g., a vascular graft, to regenerate and/or repair a tissue *in vivo, in vitro* or *ex vivo.*

Thus, one advantage of the present invention is that it provides a convenient, relatively low-cost system for sterilely isolating, seeding, and incubating cells onto a scaffold without the need for a hood or specialized facility such as an ISO Class 7 room. Another advantage is that the method can be carried out quicker than conventional methods using a hood or specialized facility. Advantageously, for example, the method can be carried out in a few hours, e.g., about 5 hours or less, preferably, about 3 hours or less, even more preferably, about two (2) hours or less.

Where a range of values is provided, it is understood that each intervening value, to the tenth of the unit of the lower limit unless the context clearly dictates otherwise, between the upper and lower limit of that range and any other stated or intervening value in that stated range is encompassed within the invention. The upper and lower limits of these smaller ranges which may independently be included in the smaller ranges is also encompassed within the invention, subject to any specifically excluded limit in the stated range. Where the stated range includes one or both of the limits, ranges excluding either both of those included limits are also included in the invention.

Although any methods and materials similar or equivalent to those described herein can also be used in the practice or testing of the present invention, the preferred methods and materials are now described.

It must be noted that as used herein and in the appended claims, the singular forms "a", "and", and "the" include plural references unless the context clearly dictates otherwise. All technical and scientific terms used herein have the same meaning.

### I. Exemplary Definitions

Unless defined otherwise, all technical and scientific terms used herein have the meaning commonly understood by a person skilled in the art to which this invention belongs. The following references provide one of skill with a general definition of many of the terms used in this invention: Singleton et al., Dictionary of Microbiology and Molecular Biology (2nd ed. 1994); The Cambridge Dictionary of Science and Technology (Walker ed., 1988); The Glossary of Genetics, 5th Ed., R. Rieger et al. (eds.), Springer Verlag (1991); and Hale & Marham, the Harper Collins Dictionary of Biology (1991). As used herein, the following terms may have meanings ascribed to them below, unless specified otherwise. However, it should be understood that other meanings that are known or understood by those having ordinary skill in the art are also possible, and within the scope of the present invention. All publications, patent applications, patents, and other references mentioned herein are incorporated by reference in their entirety. In the case of conflict, the present specification, including definitions, will control. In addition, the materials, methods, and examples are illustrative only and not intended to be limiting.

In order that the present invention may be more readily understood, certain terms are first defined.

The term "mandril" can mean, but in no way limited to, a cylindrical device or tube, e.g., a metal bar, that serves as a core around which material, e.g., a matrix scaffold for seeding and growing cells, may be cast, molded, forged, bent, or otherwise shaped. In certain embodiments, the mandril described herein is open on at least one end. In additional embodiments, the mandril described herein may also contain holes or perforations along its axis (i.e., along its length).

The term "valve" can mean, but in no way limited to, a device or part of a device by which the flow of liquid, gas, or loose material may be started, stopped, or regulated, e.g., by a movable part that opens, shuts or partially obstructs one or more ports (e.g., inlet or outlet) or passageways.

The term "biocompatible" can mean, but is not limited to, a material that the body generally accepts without a major immune response, which is capable of implantation in biological systems, for example, tissue implantation, without causing excessive fibrosis or rejection reactions.

The term "biodegradable" can mean, but is not limited to, the ability of a substance or material to break down into harmless substances by the action of a living organism(s).

The term "polymer" can mean, but is not limited to, a macromolecule formed by the chemical union of five or more identical combining units called monomers. In most cases, the number of monomer is quite large and often is not precisely known. In synthetic polymers, this number may be controlled to a predetermined extent. Combinations two, three, or four monomers are called, respectively, dimers, trimers, and tetramers, and are known collectively as oligomers. Polymers may be inorganic (e.g., siloxane, sulfur chains, black phosphorus, boron-nitrogen, silicones) or organic (meaning containing carbon). Organic polymers may be natural (e.g., polysaccharides, such as starch, cellulose, pectin, seaweed gums, vegetable gums; polypeptides, such as casein, albumin, globulin, keratin, insulin, DNA; and hydrocarbons], synthetic (such as, for example, thermoplastics (e.g., unvulcanized elastomers, nylon, polyvinyl chloride, linear polyethylene, polystyrene, polypropylene, polyurethane, acrylate resins); thermosetting (e.g., vulcanized elastomers, crosslinked polyethylene, phenolics, alkyds, polyesters), and semisynthetic (e.g., cellulosics, such as rayon, methylcellulose, cellulose acetate; and modified starches)].

The term "homopolymer" can mean, but is not limited to, a natural or synthetic polymer derived from a single monomer.

The term "heteropolymer" can mean, but is not limited to, a natural or synthetic polymer derived from more than one monomer subunit (i.e., co-polymer). Unless otherwise indicated, the term "polymer" is used generally to refer to both homopolymers and heteropolymers (i.e., co-polymer) as described herein.

The term "water soluble cellulose compounds" can mean, but is not limited to, a family of cellulose compounds that are long chain macromolecules of repeating glucose units substituted to varying extents with anionic sulfate groups, which can be represented as - SO₃⁻. Molecular weights of water soluble cellulose compounds encompassed by the invention typically range from about 5 x 10⁵ to about 3 x 10⁶ g/mol. The hydroxyl groups of each glucose unit can be substituted with from one to three sulfate groups. The sulfonation imparts water solubility to the otherwise insoluble cellulose. The availability of unsubstituted hydroxyl groups provides reactive sites for crosslinking for the soluble cellulose sulfate. The negative charge of the sulfate group is balanced by the positive charge of a cationic species, typically an alkali metal cation, and preferably the sodium cation. In certain embodiments, the polymer matrix that forms the scaffold can additionally comprise a water soluble cellulose compound, e.g., NaCS.

The term "collagen" can mean, but is not limited to, any of a family of extracellular, closely related proteins occurring as a major component of connective tissue, giving it strength and flexibility. At least fourteen (14) types exist, each composed of tropocollagen units that share a common triple-helical shape but that vary somewhat in composition between types, with the types being localized to different tissues, stages, or functions. In some types, including the most common, Type I, the tropocollagen rods associate to form fibrils or fibers; in other types the rods are not fibrillar but are associated with fibrillar collagens, while in others they form nonfibrillar, nonperiodic but structured networks. Tropocollagen, the basic structural unit of collagen comprises a helical structure consisting of three polypeptide chains, each chain composed of about a thousand amino acids, coiled around each other to form a spiral and stabilized by inter- and intrachain covalent bonds. It is rich in glycine, which occurs as nearly one residue out of three, as well as proline, hydroxyproline, and hydroxylysine; the last two rarely occur in other proteins.

The term "microscale fiber" or "micron sized fiber" can mean, but is not limited to, fibers whose diameter ranges from about 1 micrometer (10⁻⁶ m) to about 1000 micrometers.

The term "nanoscale fiber" or "nano sized fiber" can mean, but is not limited to, fibers whose diameter ranges from about 1 nanometer (10⁻⁹ m) to about 1000 nanometers.

In certain embodiments, the degradable polymer is selected from the group consisting of a poly(lactic acid-glycolic acid), a poly(lactic acid), a poly(glycolic acid), a poly(orthoester), a poly(phosphazene), poly(or polycaprolactone, a polyamide, a polysaccharide, and a collagen. In a preferred embodiment, the polymer is poly(lactic acid-glycolic acid).

As used herein, the terms "poly(glycolic acid)", polyglycolide, and "PGA" are used interchangeably herein to refer to a biodegradable, thermoplastic polymer and the simplest linear, aliphatic polyester. PGA may be obtained commercially, for example, from Sigma-Aldrich.

A "polylactide" is a biodegradable polymer derived from lactic acid. Poly(lactide) or PLA exists in two stereo forms, signified by a D or L for dexorotary or levorotary, or by DL for the racemic mix. The term "PLLA" refers to the biodegradable aliphatic polyester homopolymer poly L-lactic acid. PLLA may be obtained commercially, for example, from Alkermes, Inc.

The terms poly (lactic acid-glycolic acid), poly (D,L-lactide-c-glycoside), and PLGA are used interchangeably to refer to a copolymer of polylactic acid and glycolic acid. PLGA may be obtained commercially, for example, from Alkermes, Inc.

As used herein, the term "polysaccharide" is a long-chain natural or synthetic polymer made up of linked simple sugars (monosaccharides) such as glucose and closely related molecules. Two monosaccharide molecules may be joined by a glycosidic bond to form a disaccharide, as, for instance, in the linkage of glucose and fructose to create sucrose. More complicated polysaccharides such as starch, glycogen, cellulose or chitin consist of numerous monosaccharide units joined by glycosidic bonds.

As used herein, the term "porous" relates to having one or more openings, pores, perforations or holes that may be filled or perfused by a liquid and/or a gas, or that allows for the flow of a liquid and/or gas therethrough.

The term "growth factor" refers generally to bioactive cell signaling molecules, including cytokines and chemokines, which are known to elicit physiological effects through their interaction with cell surface receptors (typically receptor tyrosine kinases, Ser/Thr kinases, immunoglobulins or GPCRs) on a cell. The phyiological effects of growth factor binding to its receptor include, for example, changes in gene expression, and/or cell proliferation, differentiation, activation, quiescence, or apoptosis. In certain cases, growth factors are pleiotropic, i.e., they may induce different physiological effects depending on the concentration, cell type, and/or cell status. In any of the embodiments provided herein, the fiber, matrix, and/or scaffold may additionally include one or more growth factors to enhance, e.g., cell or tissue growth, differentiation, and/or repair.

The term "bioactive" and "bioactivity" can mean, but is in no way limited to, any effect on, interaction with, or response from living tissue.

As used herein, the term "fluid" can mean, but is in no way limited to, a material and/or a combination of materials capable of flowing. For example, a fluid for use in any of the embodiments described herein may include a liquid component and/or a non-liquid component (e.g., gas, solid, semi-solid, particulate, colloid) such as in a solution, a suspension, a dispersion or a combination thereof. By way of further example, the phrase "cellular isolate fluid" comprises a combination of a liquid component and a cellular or tissue-derived material.

The following embodiments of the present invention will be described in the context of a system, including an apparatus and method, for seeding, culturing, storing, shipping, and testing vascular grafts, although those skilled in the art will recognize that the disclosed methods and structures are readily adaptable for broader application. Note that whenever the same reference numeral is repeated with respect to different figures, it refers to the corresponding structure in each such figure.

### II. Exemplary Systems

**FIG. 1** illustrates an exemplary closed system **1000** provided by the invention for seeding, culturing, storing, shipping and/or testing cells and/or grafts, e.g., tissue grafts.

In certain embodiments, the system includes a means for containing a cellular isolate, e.g., a cellular isolate fluid. In certain embodiments the means is a container **3** such as a media bag or any flexible or rigid container capable of being sterilized and/or that is hermetically sealed. For example, a Gibco-BFL 1L media bag could be used. In certain embodiments, cellular isolate fluid container **3** may be composed of any biocompatible, rigid material capable of being sterilized such as Teflon, polycarbonate, PVC, or stainless steel. Container **3** can have any suitable volume for containing the cellular isolate fluid.

In a preferred embodiment, the container **3** has at least one port adapted for the sterile filling and/or dispensing of a fluid, for example, a bone marrow aspirate. For example, a bone marrow aspirate (e.g., 5cc/kg body weight) is aseptically collected and passed, e.g., injected, into container **3** via port **1** or port **2.** In another preferred embodiment, the container **3** has at least one inlet and one outlet. In still another embodiment, the container **3** includes a port having one or more valves to allow for the one-way flow of a fluid or gas. For example, using the embodiment shown in **FIG. 1** for reference, port **1** can include and/or take the form of a swabbable valve, such as a needleless access port. The valve can be connected to container **3,** and/or can be associated with a fluid line communicating with the container **3,** using any suitable coupling or fastening means which is known to those in the art, e.g., a clamp, screw or luer connector, pressure fitting, friction fitting, coupling or the like. In accordance with the embodiment of the system **1000** illustrated in **FIG. 1****,** valve **5** is associated with a conduit, e.g., a fluid line **51,** communicating with the container **3.** Optionally, the container **3** includes a pre-filter element **4,** e.g., comprising, for example, a screen or woven element having openings or a pore structure in the range of, for example, about 40 to about 150 microns. Such pre-filter element could be used to remove undesirable material such as, for example, bone chips, clots, and/or fat deposits, as the fluid passes from the container.

In certain embodiments, the means for containing a cellular isolate fluid comprises a body defining a sterile and/or hermetically sealed container capable of retaining a fluid or gas, wherein the body also defines one or more ports adapted for the filling and/or dispensing of a fluid. In certain embodiments, the body defines one or more ports, wherein at least one port has a valve, e.g., a one-way valve. In still another embodiment, the body defines an outlet port which includes a valve.

Examples of fluid which may be used in the system include, but are not limited to, sterilizing fluid, contrast media fluid, biological fluid, fluid containing cells, blood, serum, bone marrow aspirate, or fluid containing a culture medium. It is to be understood that during testing, seeding, and culturing in a preferred embodiment, the fluid may be advantageously kept at human body temperature, and may be composed of a fluid which approximates the viscosity of human blood. One illustrative example of a solution which approximates the viscosity of blood is saline with glycerol.

The fluid contained in container **3** is passed from the container through fluid line **51** (in **FIG. 1**). In a preferred embodiment, the fluid is directed away from container **3** by gravity. However, as one of skill in the art would readily appreciate, a fluid pump could also be used (e.g., Masterflex L/S Digital Drive peristaltic pump manufactured by Cole-Palmer, although one skilled in the art could select from a variety of commercially available pumps).

Fluid line **51,** as well as all other fluid lines in the system (e.g., lines **52, 53, 54, 55, 56, 57, 58a-58d,** and **59**), may be made of any type of medical grade, sterilizable, durable tubing suitable for transporting the fluid or gas in use. For example, the fluid line can be flexible or rigid plastic.

The system also includes a flow channel **7** comprising at least one inlet, at least one outlet and at least one filter comprising at least one filter medium (e.g., disposed in a filter housing) therebetween. In a preferred embodiment, the filter is disposed at an angle that is approximately perpendicular to the direction of flow through the flow channel **7** (although in some embodiments, the filter can be disposed at an angle approximately parallel to the direction of flow, e.g., involving tangential flow filtration). In preferred embodiments the filter is adapted to allow flow therethrough in at least two directions, for example, where the first and second directions are approximately opposite, e.g., wherein a fluid can be passed in a first direction from the upstream surface of the filter through the downstream surface, and a fluid can be passed in a second direction from the downstream surface of the filter though the upstream surface. In an example of this embodiment, a cellular isolate fluid is passed in a first direction through a filter having a suitable pore size (or mesh size), wherein the filter medium is at an angle that is approximately perpendicular to the direction of flow such that the filter retains cells and/or biological material that is too large to pass through the filter. A second fluid is subsequently passed in a second direction through the filter which can wash the retained cells and/or biological material off of the filter medium. Filters that can be employed for use in the flow channel are well known in the art and include, for example, Pall Corporation. In still additional embodiments, the filter (e.g., at least one filter medium) has a porosity suitable to retain cells, e.g., bone marrow-derived mononuclear cells. In certain embodiments, the filter comprises a matrix that is designed to reversibly bind and retain the cells of interest based upon, for example, ligand-receptor interactions.

In still additional embodiments, multiple filters can be assembled in series or in parallel for use in the system as described herein.

It is contemplated that the system and method can have any number of desired flowpaths and shutoffs. The liquid or gas can be fed through the system in at least three ways: gravity, such as a IV type bag connected to the filter, positive pressure or negative pressure, such as via a syringe, pump, or vacuum source, all of which are expressly encompassed and contemplated by the present invention.

Connecting to a filter and/or any other component of the system can be done in any number of ways well known in the art, for example, sterile docking, twist on fittings, such as a luer fitting, snap or friction fittings and fasteners, etc. Thus, it would be possible to connect with a variety of standard connection techniques and it is contemplated that any combination of standard connections is possible.

All combinations are encompassed and contemplated by the present invention.

In certain embodiments, the system includes a means for containing a collection fluid . In certain embodiments the means is a container **13,** such as a media bag or any flexible or rigid container capable of being sterilized and/or that is hermetically sealed. For example, a Gibco-BFL 1L media bag could be used. In certain embodiments, collection container **13** may be composed of any biocompatible, rigid material capable of being sterilized such as Teflon, polycarbonate, acrylic, PVC, or stainless steel. Container **13** can have any suitable volume for containing the collection fluid.

In a preferred embodiment, the container **13** has at least one port adapted for the sterile filling and/or dispensing of a fluid, for example, a bone marrow aspirate filtrate or flow through. For example, a bone marrow aspirate (e.g., 5cc/kg body weight) is aseptically collected and passed, e.g., injected, into container **3** and subsequently passed (e.g., through optional pre-filter **4** and via fluid flow lines **51** and **52**) through flow channel **7** including a filter. The filter retains cells and/or the biological material of interest, allowing the filtrate to flow through fluid lines **53** and **54** and inlet port **11** into container **13.** In another preferred embodiment, the container **13** has at least one flow port, e.g., a bi-directional flow port; typically, however, the container **13** has at least two ports. In certain embodiments, container **13** comprises an inlet port and/or an outlet port (in the embodiment illustrated in **FIG. 1****,** container **13** comprises an inlet port **11** and an outlet port **14**). In still another embodiment, the container **13** includes a port having one or more valves to allow for the one-way flow of a fluid or gas. The valve can be connected to container **13** and/or associated with a fluid line communicating with container **13** using any suitable coupling or fastening means which is known to those in the art, e.g., a clamp, screw or luer connector, pressure fitting, friction fitting, or the like. In accordance with the embodiment illustrated in **FIG. 1****,** the system **1000** includes a valve **10** associated with the fluid line **54.**

In certain embodiments, the means for containing the collection fluid comprises a body defining a sterile and/or hermetically sealed container capable of retaining a fluid or gas, wherein the body also defines one or more ports adapted for the filling and/or dispensing of a fluid. In certain embodiments, the body defines one or more ports, wherein at least one port has a valve, e.g., a one-way valve. In still another embodiment, the body defines an outlet port which includes a valve.

In certain embodiments (e.g., after elution fluid is passed through the flow channel 7 and cells are passed into seeding container **18** as noted in more detail below), the collection fluid or filtrate contained in container **13** is passed through fluid lines **59** and **57** into seeding container **18** (in **FIG. 1**). In a preferred embodiment, the fluid is directed away from container **13** by gravity. However, as one of skill in the art would readily appreciate, a fluid pump could also be used (e.g., Masterflex L/S Digital Drive peristaltic pump manufactured by Cole-Palmer, although one skilled in the art could select from a variety of commercially available pumps). In certain embodiments, the system includes a means for containing an elution fluid. In certain embodiments, the means is a container **9,** such as a media bag, syringe, or any flexible or rigid container capable of being sterilized and/or that is hermetically sealed. For example, a Gibco-BFL 1L media bag could be used. In certain embodiments, elution fluid container **9** may be composed of any biocompatible, rigid material capable of being sterilized such as Teflon, polycarbonate, acrylic, PVC, or stainless steel. Container **9** can have any suitable volume for containing the elution fluid.

In a preferred embodiment, the container **9** has at least one port adapted for the sterile filling and/or dispensing of a fluid, for example, an elution or washing fluid. In certain embodiments, the elution fluid can be a cell culture media, saline, e.g., phosphate buffered saline, saline including dextran, or any other suitable fluid known by those of skill in the art for harvesting or culturing cells, e.g., lactated ringers solution, normal saline, Delbecco's modified Eagles medium, a fluid as disclosed in International Publications WO 98/045413 and WO 05/094914, etc.. In another preferred embodiment, the container **9** has at least one flow port, e.g., a bi-directional flow port. In certain embodiments, container **9** comprises an inlet and/or an outlet. In still another embodiment, the container **9** includes a port having one or more valves to allow for the one-way flow of a fluid or gas, and/or one or more valves is associated with a fluid line communicating with the container **9.** In accordance with the embodiment illustrated in **FIG. 1****,** the system **1000** includes a valve **8** associated with the fluid line **55.** The valve can be connected to container **9** and/or associated with the fluid line using any suitable coupling or fastening means which is known to those in the art, e.g., a clamp, screw or luer connector, pressure fitting, friction fitting, or the like. The elution or wash fluid is passed through valve **8** and fluid line **53,** flow channel **7,** fluid lines **52, 56,** and **57,** into seeding container **18** (in **FIG. 1**).

In certain embodiments, the means for containing the elution or wash fluid comprises a body defining a sterile and/or hermetically sealed container capable of retaining a fluid or gas, wherein the body also defines one or more ports adapted for the filling and/or dispensing of a fluid. In certain embodiments, the body defines one or more ports, wherein at least one port has a valve, e.g., a one-way valve. In still another embodiment, the body defines an outlet port which includes a valve.

In one embodiment, container **9** is a syringe filled with a sterile elution or wash fluid. The elution or wash fluid is passed through flow channel **7** and into seeding container **18** through valve **8,** fluid lines **53, 52, 56** and **57** (in **FIG. 1**). In this embodiment, the fluid is directed away from container **9** due to pressure exerted on the fluid by depressing the syringe plunger, for example, manually or via a mechanical and/or electrical device. Alternatively, for example, the container **9** can be a flexible container that can be compressed. However, as one of skill in the art would readily appreciate, a fluid pump could also be used (e.g., Masterflex L/S Digital Drive peristaltic pump manufactured by Cole-Palmer, although one skilled in the art could select from a variety of commercially available pumps).

In certain embodiments, the system includes a means for containing a cell seeding assembly. In certain embodiments, the means is a seeding container **18,** such as a media bag or any flexible or rigid container capable of being sterilized and/or that is hermetically sealed. For example, a Gibco-BFL 1L media bag could be used. In certain embodiments, container **18** may be composed of any biocompatible, rigid material capable of being sterilized such as Teflon, polycarbonate, acrylic, PVC, or stainless steel. Seeding container **18** can have any suitable volume.

In certain embodiments, seeding container **18** may be comprised of two or more sections which are secured and made leak proof through any standard means, such as inner and outer threads or the use of bonding agents. For example, in accordance with the embodiment illustrated in **FIG. 1****,** the seeding container **18** comprises a rigid material comprising a main body section including threads, and a threaded cap **17.** Alternatively, in accordance with the embodiment of the seeding container **18** shown in **FIG. 1A****,** the seeding container comprises a flexible material such as a bag. In order to view the scaffold or graft, e.g., vascular graft, within container **18,** a viewing port may be placed at any point or location on the container, or alternatively, the container may be made of an optically clear material such as polycarbonate or PVC.

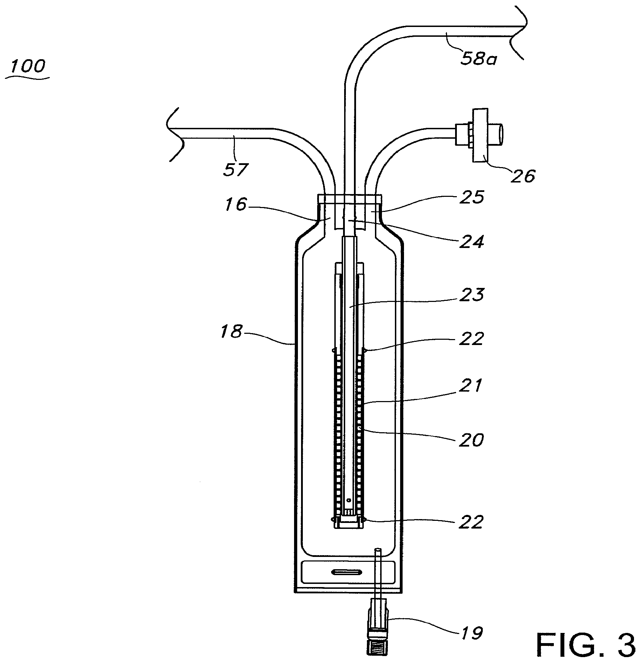

In a preferred embodiment, the seeding container **18** has at least one port adapted for the sterile filling and/or dispensing of a fluid, for example, an elution or wash fluid and/or collection or filtrate fluid as described herein. In certain embodiments, container **18** comprises an inlet and/or an outlet. In still another embodiment, the container **18** comprises at least an inlet and an outlet port (in the embodiments illustrated in **FIG. 1** and **FIG. 3****,** seeding container **18** comprises an inlet port **16,** an outlet port **24,** a sampling port **19** (e.g., for aseptic acquisition of fluid samples from the seeding container to determine, for example, microbial contamination and/or stem cell enumeration), and a vent port **26,** wherein the cap **17** (**FIG. 1** only) comprises the ports). Using the embodiments shown in **FIG. 1** and **FIG. 3** for reference, port **19** can include a swabbable valve, such as a needleless access port. In a particularly preferred embodiment, the outlet port is adapted with a cell seeding assembly **100** (see **FIG. 3**). In certain embodiments, the container **18** comprises an inlet port having one or more valves to allow for the one-way flow of a fluid or gas. Alternatively, or additionally, one or more valves can be associated with one or more fluid lines communicating with the seeding container. The valve can be connected to container **18** and/or associated with a fluid line communicating with seeding container **18** using any suitable coupling or fastening means which is known to those in the art, e.g., a clamp, screw or luer connector, pressure fitting, friction fitting, or the like.

In a preferred embodiment, seeding container **18** comprises an inlet port **16** and outlet port **24,** which allows for the perfusion and/or circulation of fluid into and through the container. Inlet port **16** and outlet port **24** are also used to attach container **18** to fluid lines **57** and **58a,** respectively. Fluid line **58a** connects seeding container **18** to one or more residual seeded cell fluid containers **35a** and **35b,** while maintaining a closed system. It is to be understood that although only one seeding container **18** is shown in **FIG. 1****,** a fluid line, e.g., fluid line **57** or **58a,** may be branched so as to connect more than one seeding container in parallel to the system.

Optionally, the seeding container **18** can further comprise at least one vent, e.g., comprising at least one hydrophobic microporous membrane (preferably, disposed in a housing) as disclosed in, for example, International Publication WO 91/017809. For example, in the embodiments illustrated in **FIG. 1** and **FIG. 3****,** a vent **26,** preferably, providing a bacterial blocking pore rating, can be placed in communication with at least one seeding container port **25.** Without being bound to any particular theory or mechanism, the vent may allow for gas exchange, e.g., while the seeded scaffold is bathed.

In certain embodiments, the means for containing the seeding assembly comprises a body defining a sterile and/or hermetically sealed container capable of retaining a fluid or gas, wherein the body also defines one or more ports adapted for the filling and/or dispensing of a fluid, and a seeding assembly. In certain embodiments, the body defines one or more ports, wherein at least one port has a valve, e.g., a one-way valve. In still another embodiment, the body defines an outlet port.

In certain embodiments, the means for containing residual seeded cell fluid is at least one residual seeded cell fluid container **35a, 35b,** such as a media bag or any flexible or rigid container capable of being sterilized and/or that is hermetically sealed. For example, a Gibco-BFL 1L media bag could be used. In certain embodiments, containers **35a, 35b** may be composed of any biocompatible, rigid material capable of being sterilized, such as Teflon, polycarbonate, PVC, or stainless steel.

In a preferred embodiment, fluid is drawn out of container **18** into a residual seeded cell fluid container **35a** via port **25** and fluid line **58a** through the use of vacuum assembly comprising a vacuum source, e.g., a pump, and a regulator **28,** wherein the negative pressure from the pump is conveyed through fluid lines connected to residual seeded cell fluid container **35a, 35b** and seeding container **18.**

In certain embodiments, seeding container **18** houses a seeding assembly **100** comprising a porous tube **20** and a scaffold **21,** e.g., cell or tissue scaffold or graft, such as a vascular graft scaffolding. The porous tube **20** may be comprised of any suitable rigid material, such as Teflon, PVC, polycarbonate, plastic, metal, e.g., stainless steel, which may be made fluid permeable. One illustrative example of a suitable porous tubing is the porous plastic tubing manufactured by Porex Technologies. Alternatively, porous tube **20** may be comprised of any suitable elastomeric material, such as PET or angioplasty balloons, that is capable of expanding and contracting, and that may be made fluid permeable. Seeding container **18** and tube **20** may both be made any length or diameter so as to hold vascular graft scaffolding **21** of any length or diameter. This is advantageous, as the system may be used to sterilize, seed, culture, store, ship, and test vascular grafts of any size. One or more retaining elements such as clips, o-rings, or grommets may also be placed on tube **20,** e.g., at both ends of scaffolding **21** ,to hold the scaffolding in place on the tube during seeding, culturing, storing, shipping, or treatment.

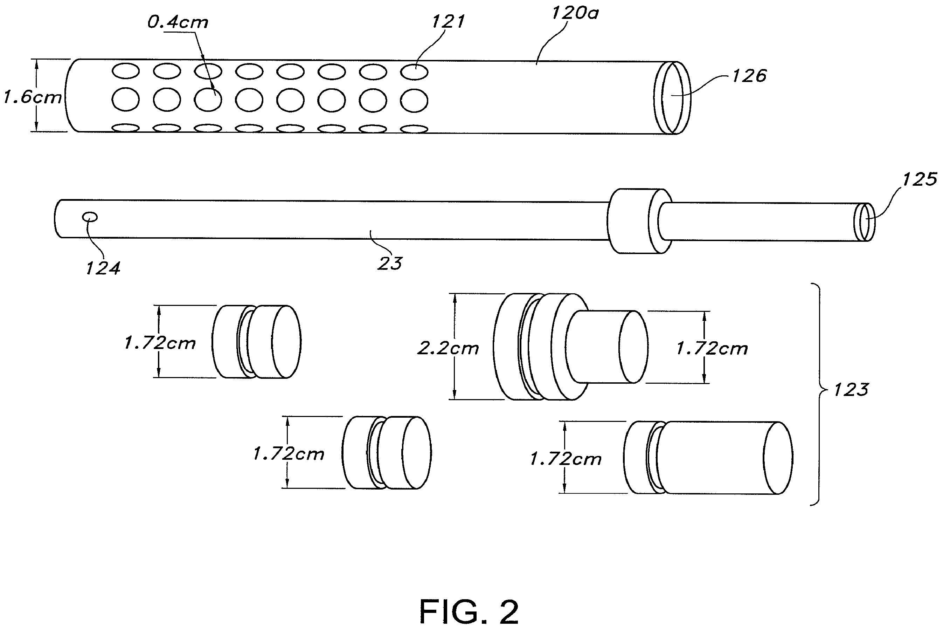

In certain embodiments, the porous tube **20** comprises a mandril **120a.** An exemplary mandril is illustrated in **FIG. 2****.** With reference to **FIG. 2****:** the open end **126** of the mandril manifold **120** is inserted over the suction rod **23** having an aperture **124** near the closed end of the suction rod. The mandril is then affixed, e.g., with appropriate sized coupling rings **123,** which are then inserted over the perforated mandril, and, illustratively, the mandril **120a** is frictionally held in the seeding container **18** via one or more o-rings **22** (shown in **FIG. 1**). This assembly allows for the movement of fluid through the mandril manifold **120** into the aperture **124** and out opening **125.** As described herein, fluid is directed out of the seeding container **18** via suction rod **23** through the mandril via a vacuum means (for example a pump) communicating with residual seeded cell fluid containers **35a, 35b,** and to the mandril **120a** through fluid lines **58a-58d** .

In a preferred embodiment, the mandril **120a** comprises a plurality of holes or perforations **121.** However, it should be understood that the perforations may be of any desired size, shape, and/or configuration, which can be varied in any number of ways that would be obvious to the skilled artisan in view of the present description, and are encompassed and contemplated by the present invention. It is also contemplated that the porous tube **20** can be of any desired length and/or diameter. For example, the diameter may be varied to account for different graft sizes and/or applications, which are expressly encompassed and contemplated by the present invention.

In certain embodiments, the seeding container **18** houses a seeding assembly **100** comprising a porous tube **18** and a scaffold **21.** The scaffold can be naturally derived, e.g., placental tissue, or synthesized. For example, synthetic cell and/or tissue scaffolds are known in the art and are commercially available. One suitable example of a scaffold is disclosed in International Publication WO 09/019995. In certain embodiments, the scaffold to be used can be a three-dimensional matrix formed of polymeric (homopolymer and/or copolymer) fibers that are assembled in a woven or non-woven mesh, in random or aligned configurations. In a preferred embodiment, the fiber matrix of the scaffold comprises pores of a suitable size to allow cells to adhere and grow and/or differentiate. Since the diameter of a cell is approximately 10 µm to 20 µm, pore sizes within this range are desired in certain embodiments. In addition, the polymeric scaffolds provided by the invention can be generated or fabricated in order to more closely mimic the structure and composition of the natural extracellular matrix in order to promote growth and differentiation of the seeded cell and to facilitate transplantation and/or implantation of the scaffold or cells grown on the same. The fibers comprising the scaffold matrix can be of any desired size, but generally are between about 1.5 mm and 1 nm. In certain embodiments, the fibers are nanoscale (i.e., from about 1 nm to about 1000 nm) and/or microscale (from about 1 µm to about 1000 µm). In certain embodiments, the scaffolds of the invention additionally comprise one or more growth factors capable of facilitating cell growth and/or differentiation.

Polymers useful for creating a scaffold for use in the present invention may be inorganic (e.g., siloxane, sulfur chains, black phosphorus, boron-nitrogen, silicones) or organic (meaning containing carbon). Organic polymers may be natural [e.g., polysaccharides, such as starch, cellulose, pectin, seaweed gums, vegetable gums; polypeptides, such as casein, albumin, globulin, keratin, collagen, insulin, DNA; and hydrocarbons], synthetic [such as thermoplastics (unvulcanized elastomers, nylon, polyvinyl chloride, linear polyethylene, polystyrene, polypropylene, polyurethane, acrylate resins); thermosetting (e.g., vulcanized elastomers, crosslinked polyethylene, phenolics, alkyds, polyesters), and semisynthetic (e.g., cellulosics, such as rayon, methylcellulose, cellulose acetate; and modified starches)]. In addition, scaffolds useful in the present invention may comprise hydrogels formed from water soluble or water insoluble cellulose compounds. As would be readily understood by the skilled artisan, the particular type and composition of scaffold will vary depending upon the desired application. However, it is generally preferred that the polymeric material comprising the scaffold be biocompatible (i.e., will not elicit an unwanted immune reaction).

In certain embodiments, the scaffold is biodegradable. In any embodiment, the degradable polymer is selected from the group consisting of a poly(lactic acid-glycolic acid), a poly(lactic acid), a poly(glycolic acid), a poly(orthoester), a poly(phosphazene), poly(or polycaprolactone, a polyamide, a polysaccharide, and a collagen. In a preferred embodiment, the polymer is poly(lactic acid-glycolic acid).

In any of the embodiments described herein, the scaffold can be apposed to the porous tube, e.g., a mandril as described herein, in the seeding assembly. For example, it is contemplated that the scaffold may be in contact with only a portion of the porous tube. Alternatively, it is contemplated that the scaffold may substantially surround some or all of the porous tube. In general, it is preferred that the scaffold be in juxtaposition or adjacent to the perforated portion of the porous tube such that fluid flows through as much of the scaffold as possible to facilitate seeding of as many cells as possible.

In accordance with embodiments of the invention, a plurality of seeding containers, e.g., comprising seeding assemblies comprising porous tubes (preferably, mandrils and scaffolds) can be pre-assembled, e.g., for different size grafts and/or different applications, and assembled as part of the system when desired. Typically, for example, the seeding containers are pre-assembled and sterilized, and can be sterilely connected to the rest of the system, e.g., by sterile docking. Thus, the optimal system can be quickly set up when needed.

In another preferred embodiment, the flow channel is positioned between the cellular isolate container, collection fluid container, elution or wash fluid container, and the seeding container. This configuration is exemplified by Figure 1. In still another embodiment, the flow channel is selectively in fluid communication with each of the same. In an embodiment, a housing comprises the flow channel, including an inlet, outlet, and a filter comprising at least one filter medium, e.g., a porous leukocyte depletion medium (for example, in a preferred embodiment, the flow channel comprises filter device comprising a housing having an inlet and an outlet and defining a fluid flow path between the inlet and an outlet, and a filter comprising at least one porous filter medium disposed in the housing across the fluid flow path). In another embodiment the housing comprises the flow channel, inlet, outlet, and filter therebetween, and a valve. In certain embodiments, the valve has at least one open position and at least one closed position.

In any of the preferred embodiments, the system provided by the invention comprises flow lines that permit the flow of a fluid and/or gas therethrough.

Accordingly, in a preferred embodiment, the invention provides a system for the seeding, culture, storage, shipping, and/or testing of a cell or tissue graft comprising a cellular isolate fluid container; a flow channel disposed between the cellular isolate fluid container, a collection fluid container, an elution fluid container, and a seeding container, wherein the flow channel comprises an inlet, an outlet and a filter therebetween, wherein the filter is adapted to allow flow in at least two directions, and wherein the flow channel is selectively in fluid communication with each of the cellular isolate fluid container; the collection fluid container, the elution fluid container, and the seeding container, respectively; and wherein the seeding container comprises a seeding assembly.

In one embodiment, the seeding assembly comprises a perforated mandril, and a biocompatible three-dimensional scaffold which is apposed to at least a portion of the mandril.

In still another embodiment, the system comprises at least one residual seeded cell fluid container, wherein the residual seeded cell fluid container is selectively in fluid communication with the seeding container.

In another embodiment, the system comprises a vacuum source in fluid communication with the residual seeded cell fluid container.

The exemplary embodiments of system as described herein (see **FIGS. 1****,****2** and **3**) allow for the isolation of bone marrow-derived mononuclear cells, and, while maintaining an aseptic system, or while maintaining a closed sterile system, seeding the cells onto a biocompatible three-dimensional scaffold, which after a brief period of incubation (e.g., about three (3) hours or less, more preferably, about two (2) hours or less) can be used as a tissue engineered vascular graft.

As would be recognized by one of skill in the art, in accordance with embodiments of the invention, the system can be used while maintaining an aseptic system, wherein a sterile seeding container can be assembled (e.g., with the desired porous tube and scaffold) using aseptic techniques, in a sterile field, such as the operating room, using sterile gloves to handle the components. The assembled seeding container can be connected to the other components of the system, e.g., wherein the cell isolation container, elution container, and collection container have already been pre-assembled in a closed sterile manner.

Alternatively, and preferably, the system can be used while maintaining a closed sterile system, wherein the system has been pre-assembled and sterilized before use.

In certain embodiments, the system is disposable. The closed disposable system allows for a procedure for the construction of tissue engineered graft, e.g., a vascular graft, that can be performed rapidly while achieving similar seeding efficiency as compared to previously described methods. See, e.g., Matsumura G, Hibino N, Ikada Y, Kurosawa H, Shinoka T. Successful application of tissue engineered vascular autografts: clinical experience. Biomaterials 2003; 24:2303-8; and FDA IDE 14127. In addition, the use of the system as described herein offers the opportunity to construct the tissue engineered graft, e.g., vascular graft, at the point of care (i.e., in the operating room precluding the need for scaffold transport.

The aseptic or closed system for seeding cells onto a three dimensional scaffold as described herein combines filter cell isolation, vacuum seeding, and container (e.g., bag) technology for use in tissue engineering in a way not previously described. Advantages of this technology include that it enables the assembly of a tissue engineered construct without the need for sterile hood or ISO Class 7 room dramatically reducing the cost for producing tissue engineered products while simultaneously increasing the clinical utility of the use of the tissue engineered product by precluding the need for such equipment or facilities. Another advantage is that it provides a graft in less time that previously available.

### III. Exemplary Methods

Tissue engineered vascular graft with bone marrow derived mononuclear cells may be obtained according to exemplary methods of the present disclosure.

With reference to **FIG. 1****,** in an additional aspect, the invention provides methods for seeding cells onto a scaffold comprising, for example, providing the cell seeding system according to any of the methods described herein, wherein at least one valve is disposed between the flow channel and each of the cellular isolate fluid container, collection fluid container, elution fluid container, and the seeding container, respectively, and wherein a first phase is defined by the flow channel being in fluid communication only with the cellular isolate fluid container and the collection fluid container; directing flow of fluid in a first direction from the cellular isolate fluid container to the collection fluid container, wherein the cells from the cellular isolate fluid are retained on the filter; closing at least one valve such that the flow channel is not in fluid communication with the collection fluid container or the cellular isolate fluid container, wherein a second phase is defined by the flow channel being in fluid communication only with the elution fluid container and the seeding container; directing flow of the elution solution from the elution fluid container through the flow channel filter in a direction approximately opposite from the first direction, such that the cells are substantially removed from the filter and flow into the seeding container; and removing the fluid from the seeding container by directing flow through the biocompatible three-dimensional scaffold and the perforated mandril into the residual seeded cell fluid container, whereby at least a portion of the cells in the seeding container are seeded onto the scaffold.

In one embodiment, the fluid is removed from the seeding container by a vacuum, e.g., -20 mm Hg (or another suitable value less than the bubble point of the filter in the flow channel 2), which is applied until all of the cell suspension has passed through the scaffold and is collected in the residual seeded cell fluid containers **35a, 35b** .

In another embodiment, a housing comprises the flow channel, including an inlet, outlet, and filter. In another embodiment the housing comprises the flow channel, inlet, outlet, and filter therebetween, and a valve. In certain embodiments, the valve has at least one open position and at least one closed position.

Accordingly, in another embodiment, and using the illustrative system **1000** shown in **FIG. 1** (wherein the seeding assembly **18** shown in **FIG. 3** can also be used in the system) for general reference, the method comprises collecting a bone marrow aspirate (e.g., 5cc/kg body weight), aseptically, into container **3,** wherein at least valve **51** is closed (typically, one or more of valves, **6, 7, 8, 10,** and **15** are also closed). Clamps **5** and **10** are opened, and, using gravity, the bone marrow aspirate is passed (via fluid lines **51** and **52**) through the flow channel **7** filter, which traps the bone marrow derived-mononuclear cells. The remaining portion of the bone marrow aspirate (typically, composed primarily of plasma) is collected (via fluid lines **53** and **54** and port **11**) in collection fluid container **13.** Subsequently, clamps **5** and **10** are closed, valves **8** and **6** are opened, and the elution solution is passed (via fluid lines **55** and **53**) through the filter **7** releasing the bone marrow-derived mononuclear cells which pass through fluid lines **52, 56,** and **57** and port **16** and are collected in the seeding container **18.** The seeding container **18** contains the seeding assembly **100,** including the scaffold **21** that is inserted over a perforated porous tube **20**/mandril **120a.** In a preferred embodiment, the bone marrow-derived mononuclear cell suspension fills the seeding container **18** completely covering the scaffold 21 that is inserted over the perforated mandril **20** (**FIG. 1A** shows the cell suspension in contact with the scaffold). Subsequently, valves **6** and **10** are closed, and valve **27** is opened, and a vacuum (e.g., -20 mm Hg) using a vacuum assembly including a regulator **28,** is applied until all of the cell suspension has passed through the scaffold and is collected, via port **24** and fluid line **58a** in residual seeded cell fluid container **35a** (if there is excess fluid, the additional cell suspension is collected, via fluid line **58b,** in residual seeded cell container **35b**).

After vacuum has ceased, valve **27** is preferably closed, valve **15** is opened, and the serum in container **13** is allowed to drain by gravity via fluid lines **59** and **57** and port **16** into seeding container **18** thus bathing the seeded scaffold. If the optional vent **26** is included as part of the seeding container **18,** gas exchange may occur, e.g., during bathing. If desired, e.g., for ease of handling the seeding container and/or the seeding container components (such as the mandril and/or scaffold) while the cells are being bathed, one or more system components upstream of the seeding container **18,** such as filter **7,** elution container **9,** collection container **13,** and/or the cellular isolate container **3** can be removed (e.g., after heat sealing the appropriate fluid line) and discarded. As an additional option, the entire apparatus is placed in an incubator at, e.g., approximately 10-100% humidity, 35-37° C, with 3-5% CO₂) for approximately two hours after which the seeded scaffold can be aseptically removed from the container (e.g., after removing the cap **17** (**FIG. 1**) or after cutting open the flexible container (**FIG. 3**)) and used as a tissue engineered vascular graft.

### IV. Exemplary Kits

In an additional aspect, the invention provides kits comprising at least one container comprising a system or apparatus according to any of the embodiments described herein and directions for its use.

### V. Examples

It should be appreciated that the exemplary embodiments of the present invention should not be construed to be limited to the examples that are described herein.

### VII. Equivalents

Those skilled in the art will recognize, or be able to ascertain using no more than routine experimentation, many equivalents to the specific embodiments of the invention described herein.

It is understood that the detailed examples and embodiments described herein are given by way of example for illustrative purposes only, and are in no way considered to be limiting to the invention. For example, the relative quantities of the ingredients may be varied to optimize the desired effects, additional ingredients may be added, and/or similar ingredients may be substituted for one or more of the ingredients described.

## Claims

1. A system for seeding cells comprising:
a cellular isolate fluid container (3);
a flow channel (7) disposed between the cellular isolate fluid container (3), a collection fluid container (13), an elution fluid container (9), and a seeding container (18), wherein the flow channel (7) comprises an inlet, an outlet and a filter therebetween, the collection fluid container (13) comprises an inlet port (11) and an outlet port (14) adapted for the sterile filling and/or dispensing of fluid, the cellular isolate fluid container (3) comprises at least one port (1,2) adapted for the sterile filling and/or dispensing of fluid, the elution fluid container (9) comprises at least one port adapted for the sterile filling and/or dispensing of fluid, and the seeding container (18) comprises at least one port (16, 24) adapted for the sterile filling and/or dispensing of fluid;
wherein the filter is adapted to allow flow in at least two directions, and wherein the flow channel (7) is selectively in fluid communication with each of the cellular isolate fluid container (3), the collection fluid container (13), the elution fluid container (9), and the seeding container (18), respectively;
and wherein the seeding container (18) comprises a cell seeding assembly (100), wherein the cell seeding assembly comprises a porous tube (20) and a scaffold (21);
wherein the cellular isolate fluid container (3) is in fluid communication with the collection fluid container (13) via the flow channel (7), fluid lines (51, 52, 53, 54), and at least one valve (5, 10);
wherein the collection fluid container (13) is in fluid communication with the seeding container (18) via fluid lines (57, 59) and at least one valve (15); and
wherein the elution fluid container (9) is in fluid communication with the seeding container (18) via fluid lines (52, 53, 55, 56, 57), the flow channel (7), and at least one valve (6, 8).

2. The system of claim 1, wherein a housing comprises the flow channel (7), inlet, outlet, and filter therebetween and wherein the seeding container (18) further comprises a vent (26).

3. The system of claim 1 or 2, comprising a closed system, wherein the system further comprises at least one residual seeded cell fluid container (35a, 35b), wherein the residual seeded cell fluid container (35 a, 35b) is selectively in fluid communication with the seeding container (18); and a vacuum source (28) in fluid communication with the residual seeded cell fluid container (35a,35b).

4. The system of any one of claims 1 to 3, wherein the seeding container (18) comprises a threaded body and a threaded cap (17).

5. The system of any one of claims 1 to 3, wherein the seeding container (18) comprises a flexible bag.

6. The system of claim 1 comprising a closed system, wherein the cellular isolate fluid and the elution solution are sterile.

7. The system of any one of claims 1 to 6, wherein the porous tube (20) comprises a perforated mandril (120a), and the scaffold (21) comprises a biocompatible three-dimensional scaffold (21) which is apposed to at least a portion of the mandril (120a), wherein the biocompatible three-dimensional scaffold is biodegradable, and wherein the biocompatible three-dimensional scaffold comprises a matrix of polymer fibers, wherein the fibers comprise at least one polymer selected from the group consisting of a poly(lactic acid-glycolic acid), a poly(lactic acid), a poly(glycolic acid), a poly(orthoester), a poly(phosphazene), polycaprolactone, a polyamide, a polyvinyl polymer or co-polymer, a polysaccharide, a collagen, and combinations thereof.

8. The system of any one of claims 1 to 6, wherein the porous tube (20) comprises a perforated mandril (120a), and the scaffold (21) comprises a biocompatible three-dimensional scaffold (21) which is apposed to at least a portion of the mandril (120a), wherein the biocompatible three-dimensional scaffold is biodegradable, and wherein the biocompatible three-dimensional scaffold comprises a matrix of polymer fibers, wherein the fibers comprise poly-1-(lactide acid).

9. The system of any one of claims 1 to 8, wherein the system comprises a plurality of seeding containers (18).

10. The system of claim 9, wherein the plurality of seeding containers is selectively in fluid communication with a manifold, which is selectively in fluid communication with the flow channel, and comprises an inlet, a valve, and a plurality of outlets, and wherein the manifold is adapted to distribute fluid and gas substantially equally to each seeding container.

11. The system of claim 7 or 8, wherein the scaffold (21) is a tissue graft capable of facilitating three dimensional tissue growth on the graft.

12. The system of claim 11, wherein the tissue graft is an implantable vascular tissue graft which surrounds at least a portion of the mandril.

13. The system of any one of claims 1 to 12, wherein each of the cellular isolate fluid container (3), elution fluid container (9), collection fluid container (13), seeding container (18), and residual cell fluid container (35a, 35b) comprises an inlet and an outlet.

14. A method for seeding cells onto a scaffold (21) comprising:
providing the system of any of claims 3 to 13, wherein at least one valve (5, 6, 8, 10, 15) is disposed between the flow channel (7) and each of the cellular isolate fluid container (3), collection fluid container (13), elution fluid container (9), and the seeding container (18), respectively, and wherein a first phase is defined by the flow channel (7) being in fluid communication only with the cellular isolate fluid container (3) and the collection fluid container (13);
directing flow of fluid in a first direction from the cellular isolate fluid container (3) to the collection fluid container (13), wherein the cells from the cellular isolate fluid are retained on the filter;
closing at least one valve (10, 5) such that the flow channel (7) is not in fluid communication with the collection fluid container (13) or the cellular isolate fluid container (3), wherein a second phase is defined by the flow channel (7) being in fluid communication only with the elution fluid container (9) and the seeding container (18);
directing flow of the elution solution from the elution fluid container (9) through the flow channel filter in a direction approximately opposite from the first direction, such that the cells are substantially removed from the filter and flow into the seeding container (18); and
removing the fluid from the seeding container (18) by directing flow through the biocompatible three-dimensional scaffold (21) and the perforated mandril (120a) into the residual cell fluid container (35a, 35b), whereby at least a portion of the cells in the seeding container (18) are seeded onto the scaffold (21).

15. The method of claim 14, wherein the method includes, prior to removing the fluid in the seeding container (18):
closing a valve (8) between the flow channel (7) and the elution fluid container (9), wherein a third phase is defined by the collection fluid container (13) being in fluid communication only with the fluid lines (57, 59) and the seeding container (18); and directing flow of fluid from the collection fluid container (13) to the seeding container (18), wherein the flow of fluid in at least one of the first phase, second phase, or third phase is due to gravity and wherein the fluid is removed from the seeding container by a vacuum.

## Patentansprüche

1. System zum Aussäen von Zellen, umfassend:
einen Zellisolatfluidcontainer (3);
einen Strömungskanal (7), welcher zwischen dem Zellisolatfluidcontainer (3), einem Sammelfluidcontainer (13), einem Elutionsfluidcontainer (9) und einem Aussaatcontainer (18) angeordnet ist, wobei der Strömungskanal (7) einen Einlass, einen Auslass und ein Filter hierzwischen umfasst, wobei der Sammelfluidcontainer (13) einen Einlass-Port (11) und einen Auslass-Port (14) umfasst, welche zum sterilen Einfüllen und/oder Ausgeben von Fluid ausgebildet sind, wobei der Zellisolatfluidcontainer (3) mindestens einen Port (1, 2) umfasst, welcher zum sterilen Einfüllen und/oder Ausgeben von Fluid ausgebildet sind, wobei der Elutionsfluidcontainer (9) mindestens einen Port umfasst, welcher zum sterilen Einfüllen und/oder Ausgeben von Fluid ausgebildet ist, und wobei der Aussaatcontainer (18) mindestens einen Port (16, 24) umfasst, welcher zum sterilen Einfüllen und/oder Ausgeben von Fluid ausgebildet ist;
wobei das Filter dazu ausgebildet ist, eine Strömung in mindestens zwei Richtungen zuzulassen, und wobei der Strömungskanal (7) jeweils selektiv mit dem Zellisolatfluidcontainer (3), dem Sammelfluidcontainer (13), dem Elutionsfluidcontainer (9) bzw. dem Aussaatcontainer (18) in Fluidverbindung steht;
und wobei der Aussaatcontainer (18) eine Zellaussaatanordnung (100) umfasst, wobei die Zellaussaatanordnung ein poröses Rohr (20) und ein Scaffold (21) umfasst;
wobei der Zellisolatfluidcontainer (3) über den Strömungskanal (7), Fluidleitungen (51, 52, 53, 54) und mindestens ein Ventil (5, 10) mit dem Sammelfluidcontainer (13) in Fluidverbindung steht;
wobei der Sammelfluidcontainer (13) über Fluidleitungen (57, 59) und mindestens ein Ventil (15) mit dem Aussaatcontainer (18) in Fluidverbindung steht; und
wobei der Elutionsfluidcontainer (9) über Fluidleitungen (52, 53, 55, 56, 57), den Strömungskanal (7) und mindestens ein Ventil (6, 8) mit dem Aussaatcontainer (18) in Fluidverbindung steht.

2. System nach Anspruch 1, wobei ein Gehäuse den Strömungskanal (7), den Einlass, den Auslass und das Filter hierzwischen umfasst und wobei der Aussaatcontainer (18) ferner einen Lüftungsöffnung (26) umfasst.

3. System nach Anspruch 1 oder 2, umfassend ein geschlossenes System, wobei das System ferner umfasst: mindestens einen Container (35a, 35b) für ausgesäte Zellen enthaltendes Restfluid, wobei der Container (35a, 35b) für ausgesäte Zellen enthaltendes Restfluid selektiv in Fluidverbindung mit dem Aussaatcontainer (18) steht; und eine Vakuumquelle (28) in Fluidverbindung mit dem Container (35a, 35b) für ausgesäte Zellen enthaltendes Restfluid.

4. System nach einem der Ansprüche 1 bis 3, wobei der Aussaatcontainer (18) einen Gewindekörper und eine Gewindekappe (17) umfasst.

5. System nach einem der Ansprüche 1 bis 3, wobei der Aussaatcontainer (18) einen flexiblen Beutel umfasst.

6. System nach Anspruch 1, umfassend ein geschlossenes System, wobei das Zellisolatfluid und die Elutionslösung steril sind.

7. System nach einem der Ansprüche 1 bis 6, wobei das poröse Rohr (20) einen perforierten Dorn (120a) umfasst und das Scaffold (21) ein biokompatibles dreidimensionales Scaffold (21) umfasst, welches an mindestens einem Teil des Dorns (120a) anliegt, wobei das biokompatible dreidimensionale Scaffold biologisch abbaubar ist und wobei das biokompatible dreidimensionale Scaffold eine Matrix von Polymerfasern umfasst, wobei die Fasern mindestens ein Polymer umfassen, welches ausgewählt ist aus der Gruppe bestehend aus einer Polymilchsäure-Glycolsäure, einer Polymilchsäure, einer Polyglycolsäure, einem Polyorthoester, einem Polyphosphazen, einem Polycaprolacton, einem Polyamid, einem Polyvinyl-Polymer oder -Copolymer, einem Polysaccharid, einem Kollagen und Kombinationen hiervon.

8. System nach einem der Ansprüche 1 bis 6, wobei das poröse Rohr (20) einen perforierten Dorn (120a) umfasst und das Scaffold (21) ein biokompatibles dreidimensionales Scaffold (21) umfasst, welches an mindestens einem Teil des Dorns (120a) anliegt, wobei das biokompatible dreidimensionale Scaffold biologisch abbaubar ist und wobei das biokompatible dreidimensionale Scaffold eine Matrix von Polymerfasern umfasst, wobei die Fasern Poly-L-Milchsäure umfassen.

9. System nach einem der Ansprüche 1 bis 8, wobei das System eine Mehrzahl von Aussaatcontainern (18) umfasst.

10. System nach Anspruch 9, wobei die Mehrzahl von Aussaatcontainern selektiv in Fluidverbindung mit einer Anordnung stehen, welche selektiv in Fluidverbindung mit dem Strömungskanal steht und einen Einlass, ein Ventil und eine Mehrzahl von Auslässen umfasst, und wobei die Anordnung dazu ausgebildet ist, Fluid und Gas im Wesentlichen gleichmäßig auf jeden Aussaatcontainer zu verteilen.

11. System nach Anspruch 7 oder 8, wobei das Scaffold (21) ein Gewebetransplantat ist, welches dazu in der Lage ist, dreidimensionales Gewebewachstum auf dem Transplantat zu erleichtern.

12. System nach Anspruch 11, wobei das Gewebetransplantat ein implantierbares Gefäßgewebetransplantat ist, welches mindestens einen Teil des Dorns umgibt.

13. System nach einem der Ansprüche 1 bis 12, wobei jeder der Container Zellisolatfluidcontainer (3), Elutionsfluidcontainer (9), Sammelfluidcontainer (13), Aussaatcontainer (18) und Container (35a, 35b) für ausgesäte Zellen enthaltendes Restfluid einen Einlass und einen Auslass umfasst.

14. Verfahren zum Aussäen von Zellen auf ein Scaffold (21), umfassend:
Bereitstellen des Systems nach einem der Ansprüche 3 bis 13, wobei mindestens ein Ventil (5, 6, 8, 10, 15) zwischen dem Strömungskanal (7) und jedem der Container Zellisolatfluidcontainer (3), Sammelfluidcontainer (13), Elutionsfluidcontainer (9) bzw. Aussaatcontainer (18) angeordnet ist, und wobei eine erste Phase dadurch definiert ist, dass der Strömungskanal (7) nur mit dem Zellisolatfluidcontainer (3) und dem Sammelfluidcontainer (13) in Fluidverbindung steht;
Richten eines Fluidstroms in einer ersten Richtung von dem Zellisolatfluidcontainer (3) zu dem Sammelfluidcontainer (13), wobei die Zellen aus dem Zellisolatfluid an dem Filter zurückgehalten werden;
Schließen mindestens eines Ventils (10, 5), derart, dass der Strömungskanal (7) nicht in Fluidverbindung mit dem Sammelfluidcontainer (13) oder dem Zellisolatfluidcontainer (3) steht, wobei eine zweite Phase dadurch definiert ist, dass der Strömungskanal (7) nur mit dem Elutionsfluidcontainer (9) und dem Aussaatcontainer (18) in Fluidverbindung steht;
Richten eines Stroms der Elutionslösung von dem Elutionsfluidcontainer (9) durch das Strömungskanalfilter hindurch in einer zu der ersten Richtung annähernd entgegengesetzten Richtung, derart, dass die Zellen im Wesentlichen aus dem Filter abgezogen werden und in den Aussaatcontainer (18) strömen; und
Abziehen des Fluids aus dem Aussaatcontainer (18) durch Richten eines Stroms durch das biokompatible dreidimensionale Scaffold (21) und den perforierten Dorn (120a) hindurch in den Container (35a, 35b) für ausgesäte Zellen enthaltendes Restfluid hinein, wodurch mindestens ein Teil der Zellen in dem Aussaatcontainer (18) auf das Scaffold (21) ausgesät werden.

15. Verfahren nach Anspruch 14, wobei das Verfahren umfasst: vor dem Schritt des Abziehens des Fluids aus dem Aussaatcontainer (18):
Schließen eines Ventils (8) zwischen dem Strömungskanal (7) und dem Elutionsfluidcontainer (9), wobei eine dritte Phase dadurch definiert ist, dass der Sammelfluidcontainer (13) nur mit den Fluidleitungen (57, 59) und dem Aussaatcontainer (18) in Fluidverbindung steht; und
Richten eines Fluidstroms von dem Sammelfluidcontainer (13) zu dem Aussaatcontainer (18), wobei der Fluidstrom in mindestens einer der Phasen erste Phase, zweite Phase oder dritte Phase durch Schwerkraft erfolgt und wobei das Fluid mittels eines Vakuums aus dem Aussaatcontainer abgezogen wird.

## Revendications

1. Système pour ensemencer des cellules comprenant :
un récipient de fluide d'isolat cellulaire (3) ;
un canal d'écoulement (7) disposé entre le récipient de fluide d'isolat cellulaire (3), un récipient de fluide de collecte (13), un récipient de fluide d'élution (9), et un récipient d'ensemencement (18), lequel canal d'écoulement (7) comprend une entrée, une sortie et un filtre entre elles, lequel récipient de fluide de collecte (13) comprend un orifice d'entrée (11) et un orifice de sortie (14) adaptés pour le remplissage et/ou la distribution stériles de fluide, lequel récipient de fluide d'isolat cellulaire (3) comprend au moins un orifice (1, 2) adapté pour le remplissage et/ou la distribution stériles de fluide, lequel récipient de fluide d'élution (9) comprend au moins un orifice adapté pour le remplissage et/ou la distribution stériles de fluide, et lequel récipient d'ensemencement (18) comprend au moins un orifice (16, 24) adapté pour le remplissage et/ou la distribution stériles de fluide ;
dans lequel le filtre est adapté pour permettre un écoulement dans au moins deux directions, et dans lequel le canal d'écoulement (7) est sélectivement en communication de fluide avec chacun parmi le récipient de fluide d'isolat cellulaire (3), le récipient de fluide de collecte (13), le récipient de fluide d'élution (9), et le récipient d'ensemencement (18), respectivement ;
et dans lequel le récipient d'ensemencement (18) comprend un assemblage d'ensemencement de cellules (100), lequel assemblage d'ensemencement de cellules comprend un tube poreux (20) et une charpente (21) ;
dans lequel le récipient de fluide d'isolat cellulaire (3) est en communication de fluide avec le récipient de fluide de collecte (13) via le canal d'écoulement (7), des conduites de fluide (51, 52, 53, 54) et au moins une vanne (5, 10) ;
dans lequel le récipient de fluide de collecte (13) est en communication de fluide avec le récipient d'ensemencement (18) via des conduites de fluide (57, 59) et au moins une vanne (15) ; et
dans lequel le récipient de fluide d'élution (9) est en communication de fluide avec le récipient d'ensemencement (18) via des conduites de fluide (52, 53, 55, 56, 57), le canal d'écoulement (7), et au moins une vanne (6, 8).

2. Système selon la revendication 1, dans lequel un boîtier comprend le canal d'écoulement (7), l'entrée, la sortie et le filtre entre elles, et dans lequel le récipient d'ensemencement (18) comprend en outre une aération (26).

3. Système selon la revendication 1 ou 2, comprenant un système fermé, lequel système comprend en outre au moins un récipient de fluide cellulaire ensemencé résiduel (35a, 35b), dans lequel le récipient de fluide cellulaire ensemencé résiduel (35a, 35b) est sélectivement en communication de fluide avec le récipient d'ensemencement (18) ; et une source de vide (28) en communication de fluide avec le récipient de fluide cellulaire ensemencé résiduel (35a, 35b).

4. Système selon l'une quelconque des revendications 1 à 3, dans lequel le récipient d'ensemencement (18) comprend un corps fileté et un bouchon fileté (17).

5. Système selon l'une quelconque des revendications 1 à 3, dans lequel le récipient d'ensemencement (18) comprend un sac flexible.

6. Système selon la revendication 1 comprenant un système fermé, dans lequel le fluide d'isolat cellulaire et la solution d'élution sont stériles.

7. Système selon l'une quelconque des revendications 1 à 6, dans lequel le tube poreux (20) comprend un mandrin perforé (120a), et la charpente (21) comprend une charpente tridimensionnelle biocompatible (21) qui est apposée à au moins une partie du mandrin (120a), dans lequel la charpente tridimensionnelle biocompatible est biodégradable, et dans lequel la charpente tridimensionnelle biocompatible comprend une matrice de fibres polymères, dans lequel les fibres comprennent au moins un polymère choisi dans le groupe constitué par un poly(acide lactique-acide glycolique), un poly(acide lactique), un poly(acide glycolique), un poly(orthoester), un poly(phosphazène), une polycaprolactone, un polyamide, un polymère ou copolymère polyvinylique, un polysaccharide, un collagène, et leurs combinaisons.

8. Système selon l'une quelconque des revendications 1 à 6, dans lequel le tube poreux (20) comprend un mandrin perforé (120a), et la charpente (21) comprend une charpente tridimensionnelle biocompatible (21) qui est apposée à au moins une partie du mandrin (120a), dans lequel la charpente tridimensionnelle biocompatible est biodégradable, et dans lequel la charpente tridimensionnelle biocompatible comprend une matrice de fibres polymères, dans lequel les fibres comprennent du poly-1-(acide lactide).

9. Système selon l'une quelconque des revendications 1 à 8, lequel système comprend une pluralité de récipients d'ensemencement (18).

10. Système selon la revendication 9, dans lequel la pluralité de récipients d'ensemencement est sélectivement en communication de fluide avec un collecteur, qui est sélectivement en communication de fluide avec le canal d'écoulement, et comprend une entrée, une vanne, une pluralité de sorties, et dans lequel le collecteur est adapté pour distribuer un fluide et un gaz pratiquement également dans chaque récipient d'ensemencement.

11. Système selon la revendication 7 ou 8, dans lequel la charpente (21) est un greffon tissulaire capable de faciliter une croissance tissulaire tridimensionnelle sur le greffon.

12. Système selon la revendication 11, dans lequel le greffon tissulaire est un greffon tissulaire vasculaire implantable qui entoure au moins une partie du mandrin.

13. Système selon l'une quelconque des revendications 1 à 12, dans lequel chacun parmi le récipient de fluide d'isolat cellulaire (3), le récipient de fluide d'élution (9), le récipient de fluide de collecte (13), le récipient d'ensemencement (18) et le récipient de fluide cellulaire résiduel (35a, 35b) comprend une entrée et une sortie.

14. Procédé pour ensemencer des cellules sur une charpente (21) comprenant :
l'obtention du système de l'une quelconque des revendications 3 à 13, où au moins une vanne (5, 6, 8, 10, 15) est disposée entre le canal d'écoulement (7) et chacun parmi le récipient de fluide d'isolat cellulaire (3), le récipient de fluide de collecte (13), le récipient de fluide d'élution (9), et le récipient d'ensemencement (18), respectivement, et dans lequel une première phase est définie par le canal d'écoulement (7) qui est en communication de fluide uniquement avec le récipient de fluide d'isolat cellulaire (3) et le récipient de fluide de collecte (13) ;
la direction de l'écoulement de fluide dans une première direction depuis le récipient de fluide d'isolat cellulaire (3) vers le récipient de fluide de collecte (13), où les cellules provenant du fluide d'isolat cellulaire sont retenues sur le filtre ;
la fermeture d'au moins une vanne (10, 5) de façon que le canal d'écoulement (7) ne soit pas en communication de fluide avec le récipient de fluide de collecte (13) ou le récipient de fluide d'isolat cellulaire (3), où une deuxième phase est définie par le canal d'écoulement (7) qui est en communication de fluide uniquement avec le récipient de fluide d'élution (9) et le récipient d'ensemencement (18) ;
la direction de l'écoulement de la solution d'élution depuis le premier récipient de fluide d'élution (9) à travers le filtre de canal d'écoulement dans une direction approximativement opposée à la première direction, de façon que les cellules soient sensiblement retirées du filtre et s'écoulent dans le récipient d'ensemencement (18) ; et
le retrait du fluide hors du récipient d'ensemencement (18) par direction de l'écoulement à travers la charpente tridimensionnelle biocompatible (21) et le mandrin perforé (120a) dans le récipient de fluide cellulaire résiduel (35a, 35b), moyennant quoi au moins une partie des cellules dans le récipient d'ensemencement (18) sont ensemencées sur la charpente (21).

15. Procédé selon la revendication 14, lequel procédé comprend, avant le retrait du fluide dans le récipient d'ensemencement (18) :
la fermeture d'une vanne (8) entre le canal d'écoulement (7) et le récipient de fluide d'élution (9), où une troisième phase est définie par le récipient de fluide de collecte (13) qui est en communication de fluide uniquement avec les lignes de fluide (57, 59) et le récipient d'ensemencement (18) ; et
la direction de l'écoulement de fluide depuis le récipient de fluide de collecte (13) vers le récipient d'ensemencement (18),
dans lequel l'écoulement de fluide dans au moins l'une parmi la première phase, la deuxième phase et la troisième phase est dû à la gravité et dans lequel le fluide est retiré du récipient d'ensemencement au moyen d'un vide.
